(19)

Europäisches
Patentamt

European
Patent Office

Office européen
des brevets

(11) **EP 2 515 760 B1**

(12) **EUROPEAN PATENT SPECIFICATION**

(45) Date of publication and mention
of the grant of the patent:
**12.02.2014 Bulletin 2014/07**

(21) Application number: **09795990.2**

(22) Date of filing: **21.12.2009**

(51) Int Cl.:
***A61B 5/16*** (2006.01)

(86) International application number:
**PCT/EP2009/067641**

(87) International publication number:
**WO 2011/076243 (30.06.2011 Gazette 2011/26)**

(54) **AFFECTIVE WELL-BEING SUPERVISION SYSTEM AND METHOD**

SYSTEM UND VERFAHREN ZUR ÜBERWACHUNG VON EMOTIONALEM WOHLBEFINDEN

SYSTÈME ET MÉTHODE DE SUPERVISION DU BIEN-ÊTRE AFFECTIF

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**31.10.2012 Bulletin 2012/44**

(73) Proprietor: **FUNDACIÓN TECNALIA RESEARCH &
INNOVATION
20009 San Sebastián (Guipúzcoa) (ES)**

(72) Inventors:
• **LEÓN VILLEDA, Enrique, Edgar**
E-20009 San Sebastian (ES)
• **MONTALBÁN PONTESTA, Iraitz**
E-20009 San Sebastian (ES)
• **GARZO MANZANARES, Ainara**
E-20009 San Sebastian (ES)

(74) Representative: **Carpintero Lopez, Francisco et al
Herrero & Asociados, S.L.
Alcalá 35
28014 Madrid (ES)**

(56) References cited:
**EP-A1- 1 656 880          WO-A2-2006/090371
US-A1- 2009 253 996**

• **AINARA GARZO ET AL: "User-centred
Physiological Emotion Detection for Assistive
Technology" ASSISTIVE TECHNOLOGY FROM
ADAPTED EQUIPMENT TO INCLUSIVE
ENVIRONMENTS 20090101 IOS PRESS LNKD-
DOI:10.3233/978-1-60750-042-1-353, vol. 25, 1
January 2009 (2009-01-01), pages 353-357,
XP009133124 ISBN: 978-1-60750-042-1**
• **LEON E ET AL: "Real-time detection of emotional
changes for inhabited environments"
COMPUTERS AND GRAPHICS, ELSEVIER, GB
LNKD- DOI:10.1016/J.CAG.2004.06.002, vol. 28,
no. 5, 1 October 2004 (2004-10-01), pages 635-642,
XP004526637 ISSN: 0097-8493**
• **LEON ET AL: "A user-independent real-time
emotion recognition system for software agents
in domestic environments" ENGINEERING
APPLICATIONS OF ARTIFICIAL INTELLIGENCE,
PINERIDGE PRESS, SWANSEA, GB LNKD- DOI:
10.1016/J.ENGAPPAI.2006.06.001, vol. 20, no. 3,
13 March 2007 (2007-03-13) , pages 337-345,
XP005919099 ISSN: 0952-1976**

EP 2 515 760 B1

**Description**

**FIELD OF THE INVENTION**

This invention relates to the field of applied psychophysiology.

**BACKGROUND OF THE INVENTION - RELATED ART**

The relationship between emotions and health has been widely investigated and unequivocally evidenced. It has been confirmed that negative emotions are linked to greater risks of suffering from a number of immunological and cardiovascular diseases and can also contribute to unfavourable behavioural changes that lead to increased morbidity. The continuous reoccurrence of such unfavourable emotions in the individual may lead to the development of clinical depression and other impairing mental or affective disorders. Positive emotions on the other hand assist not only in protecting against the cardiovascular sequelae of negative emotions but also in improving surgery recovery and contributing towards enhanced longevity. It thus seems evident that by alleviating unpleasant emotions one may be able to improve the health conditions and general well-being of a person. This might bear particular relevance in old age where the adverse consequences of unpleasant affects might be further exacerbated owing to numerous emotional stressors that arise with aging such as damage to self-confidence, isolation, disability, discrimination, loss of independence, lack of mobility, fear of death, chronic illnesses, or alcoholism and other substance abuse.

Emotions have been traditionally studied using instruments like self-questionnaires, behavioural observation, projective techniques, and analysis of facial, speech or physiological parameters. The latter techniques have been in recent years adopted by technologists for the purpose of improved human-machine interaction, higher customization levels in computing systems, and emotional feedback. In facial emotion detection video cameras and image processing systems are used to identify emotions based on spatial and geometrical relationships of eyes, eyebrows and mouth. Speech emotion detection on the other hand relies on the idea that particular ways of intonation convey information about the current emotional state of the speaker. Finally, physiological emotion detection gauges the changes that affective phenomena provoke in a number of signals of the brain and autonomic nervous system (ANS) and hence shares common roots with the field of psychophysiology, an area of scientific research that investigates the relationships between physiological changes and cognitive and emotional phenomena. Although facial and speech emotion recognition methods have greatly advanced in recent years, their operation is still primarily bound to experimental settings where the stringent conditions for the acquisition of facial and speech data can be found. In contrast, the possibility of continuously collecting data while the person undertakes daily life activities is what makes physiological emotion detection more appealing to those interested in studying or detecting emotions in daily life than the facial and speech approaches.

For example, US 2003/139654 suggests the utilization of a support vector machine (SVM) classifier based on three physiological signals, namely ECG, skin conductance and temperature, to classify four emotions: sadness, anger, stress and surprise. US 6,656,116 B2 presents another physiological emotion detection system which identifies emotions based on statistical differences of mean values calculated from a number of physiological signals.

It is also known that alternative physiological measurements can be used, such as galvanic skin response (GSRe), skin temperature, and heart rate. Additionally, normalised signals can be employed instead of statistical features.

In yet another example, US 6190314B1 discloses an adaptable computer environment based on emotional information estimated from physiological signals acquired through a computer mouse. Six emotional classes are identified using data acquired from somatic activity (mouse movement), skin resistance, skin temperature, and heart rate.

A variety of physiological measurements are known to have been used to detect emotional states, such as galvanic skin response (GSRe), blood volume pressure (BVP), heart rate (HR), electromyogram (EMG), skin conductivity (SC), respiration amplitude and rate (RESP), electrocardiogram (ECG), the vertical component of the electrooculogram (EOG), the tonic and phasic element of the electrodermal activity (EDA), etc. Different mathematical approaches to treat these measurements have also been used, including statistical features in conjunction with Hidden Markov Models (HMMs), neural networks, support vector machines (SVM), dynamic batch learning vector quantization (DBLVQ) and decision trees (DT), combinations of Linear and Quadratic Discriminant Analysis, and sequential probability ratio test (SPRT).

Other approaches that illustrate prior art in emotion detection can be mentioned. US 5,507,291 relates to a method to remotely detect emotions using the amount of energy reflected by a person's body. US 2008/221401, describes a method to perform physiological emotion detections using continuous emotional stimulation in order to determine baseline values. US 5,601,090 disclose an invention to identify and quantify a number of emotional states (called somatic states) using frequency bands applied onto a neural network. US 5,676,138 outlines a multimedia system that measures, analyses, stores and display emotional responses to a number of prespecified affective stimuli using a statistical measure called the z-Score. WO 2008/129356 determines the affective state of a person simultaneously using eye properties and the visual fixation point. US 6,609,024 discloses a method to measure emotional valence using brainwave signals.

A ratio of asymmetry between left and right hemispheres brain signals over time is calculated and provided to a neural network which then determines whether a person is emotionally positive or negative. Another method to detect emotional valence using brain-signals is disclosed in US 6,021,346. On this occasion the increase or decrease over time in the relative power of a subband of a specific frequency band in Electroencephalogram (EEG) signals is used to detect emotions. US 2007/0192108 discloses a system to display an emotion based on the analysis of voice signals.

Additional inventions related to emotion detection can be found in a group of devices that belong to the area of biofeedback and physiological monitoring. Note that although the majority of biofeedback systems focuses on providing physiological information to a person for the purpose of supporting bodily changes that lead to an improvement in health, they sometimes also inform about accompanying emotional states and can thus be related to applied psychophysiology and physiological emotion detection. For example patent WO 2008/028391 describes a wearable device that measures and transmits information about the current skin temperature of a person onto and electronic communication. A medical practitioner then has to infer about the emotional state of the wearer. JP 2005/237668 introduces a system to identify emotional and physical abnormalities using a combination of facial, speech and physiological information. US 2007/0142732 discloses a method to detect heart failure decompensation using cumulative-sum-trend analysis on physiological data acquired from a number of sensors. This method can be embodied as a medical device that features a therapy control unit. US 5,974,262 discloses an interactive system that reacts to physiological responses associated with affective states. US 4,683,891 presents an interactive biomonitoring system to measure and display stress levels using a combination of physiological signals and computer inputs. A similar system is disclosed in US 5,741,217 where a system consisting of a GSR sensor and a computer employ physiological information to provide visual and/or audiofeedback to the user. US 5,682,803 discloses a wireless device to collect physiological data which may be utilized to perform medical diagnosis. A biofeedback apparatus for therapeutic purposes is presented in US 6,026,322 where visual and pictorial representations of physiological and psychological conditions are produced. The user can then regulate their body to achieve a given beneficial effect (lower anxiety levels) using such representations. US 2007/0167850 relates to an apparatus and methods to perform adaptive physiological monitoring. In this case adaptive refers to the capacity of the system to feedback back information to the user only when the physiological signals indicate that the user is in a normal activity state. WO 2009/037612A2 discusses a method to detect an abnormal situation (falls in particular) related to motion, physiological and/or environmental sensors. Similarly, application WO2006009830A2 presents a system to monitor and display physiological signals in ambulatory conditions and identify abnormal conditions. US 2009/253996 also discloses a system for emotion detection.

However, for a successful implementation of applied psychophysiology and emotion detection methodologies into technology that meets the requirements of real-world applications, e.g. affective tele-assistance, four conditions are required: the possibility of such methodologies to unrestrictedly be used by a plurality of people independent of their individual characteristics (user-independence); the capacity of such methodologies to identify emotions in real-time using flexible methodologies; the ability to do it so while the person undertakes daily-life activities; and the attribute of being adaptive to gradual physical changes while featuring long-term performance evaluation. All of the above disclosures demonstrate shortcomings in one or more of the above requirements.

Hence it seems apparent that the prior art does not offer a convenient, reliable solution to identify emotions in situations involving high mobility and ambulatory conditions like the ones associated with domestic life. On a second aspect, prior art does not solve the problem of responding to negative emotions using the persons own preferences in order to palliate the detrimental effects of said negative emotions.

## SUMMARY OF THE INVENTION

The current invention solves the aforementioned problems by disclosing a system defined in claim 1 and method defined in claim 9 that respond to an emotional negative state of a person using external devices (such as ambient electronic devices), preferably in a way that is configurable by the said person. This innovates on previous systems employing applied psychophysiology in respect to the ability of a given user to accommodate their own needs and preferences in an attempt to overcome the damaging effects of negative emotions.

In the present invention, an affective well-being supervision system is disclosed comprising:

- wearable sensors, which measure physiological signals of a user.
- logical means, configured to be connected to the sensors and to an exterior device (such as ambient electronic devices, radios, etc), and which analyze the data from the sensors and generates and sends commands to the exterior device whenever a negative emotion is detected on the measured physiological signals.

The system further inquires the person about their emotional state and uses that information to perform long-term, continuous learning. In doing so the system identifies changes in the physiology of the subject and automatically adapts its response to account for said changes. The methods to detect emotions are preferably based on autoassociative

3

memories which contrary to other techniques have been shown to resist data perturbations caused by non-emotional physiological changes caused by physical exertion and sensor faults. Because an immediate classification of said non-emotional changes might results in false positives or negatives, the present invention offers a more stable mechanism based on non-parametric sequential change point detection methodologies. In doing so the present system indicates the occurrence of an emotional state only after data from various successive data samples have been analyzed thereby reducing the impact of transitory non-emotional physiological changes. Furthermore, an emotional class is identified based on the simultaneous results from diverse classification methods thereby providing additional accuracy.

In another aspect of the present invention, a method for supervising the affective well-being of a user is disclosed. The method comprises measuring physiological signals of the user, detecting emotional changes on said signals, and generating commands for an external device in order to compensate for or alleviate said emotional change.

These and other advantages will be apparent in the light of the detailed description of the invention.

## BRIEF DESCRIPTION OF THE DRAWINGS

For the purpose of aiding the understanding of the characteristics of the invention, according to a preferred practical embodiment thereof and in order to complement this description, the following figures are attached as an integral part thereof, having an illustrative and non-limiting character:

FIG. 1 provides a schematic representation of the main components of the system.
FIG. 2 illustrates the flow of information to and from the Embedded Computer (EC).
FIG. 3 shows a schematic representation of the elements which identify emotional changes and classify said changes into a number of affective labels.
FIG. 4 outlines an example of the series of windows that constitute the EC interface.
FIG. 5 is a flowchart of the process to perform long-term adaptation of the Autoassociative Memory (AM).
FIG. 6 shows the classifier in further detail.

## DETAILED DESCRIPTION OF THE INVENTION

The matters defined in this detailed description are provided to assist in a comprehensive understanding of the invention. Accordingly, those of ordinary skill in the art will recognize that variation changes and modifications of the embodiments described herein can be made without departing from the scope and spirit of the invention. Also, description of well-known functions and elements are omitted for clarity and conciseness.

Note that in this text, the term "comprises" and its derivations (such as "comprising", etc.) should not be understood in an excluding sense, that is, these terms should not be interpreted as excluding the possibility that what is described and defined may include further elements, steps, etc.

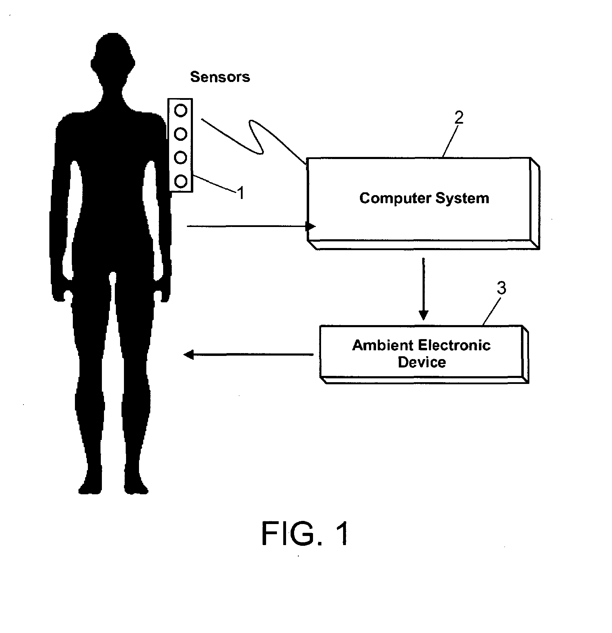

Figure 1 shows a system according to a preferred embodiment of the present inventions. A plurality of wearable sensors 1 provide measurements of physiological signals to an embedded computer system (EC) 2, which analyzes the data received from the sensors (that is, the measurements of the physiological signals) and sends commands to external electronic devices 3 (also referred as ambient electronic devices). The communications between the sensors and the EC, and between the EC and the external electronic devices are preferably wireless.

An ambient electronic device (AED) is an apparatus that receives information remotely most commonly using wireless fidelity (WiFi) although other forms of communication also exist, e.g. radiofrequency. Said information usually takes the form of a command that initiates an action determined by the user's previously expressed preferences and the tools and configurations specific to said AED, e.g. playing certain type of music, displaying text messages.

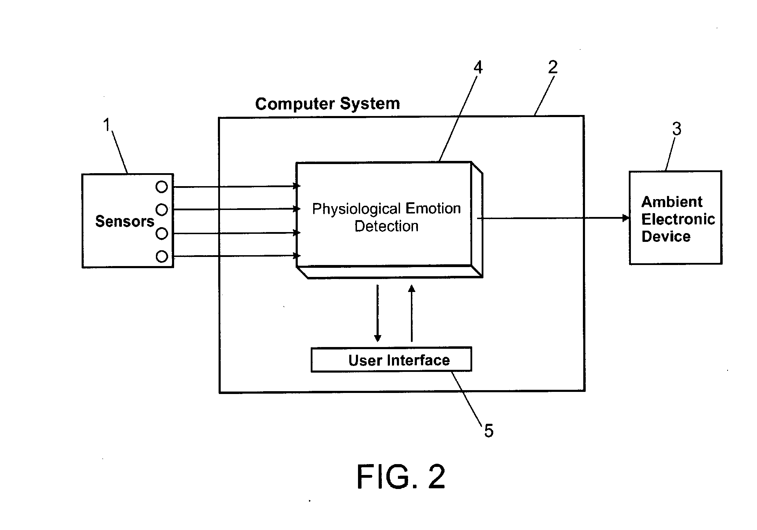

Figure 2 shows the EC 2 in further detail. The EC comprises both logical means 4 which perform the analysis and instruction generation, and a user interface 5 that allows the user to customize the generated instructions and to confirm that the detected affective states are correct. The user interface is preferably a touch screen (referred in the present document as ECTS), although other ways of communication between the user and the EC are possible, such as, for example, voice recognition. The logical means 4 are also referred as Physiological Emotion Detection System.

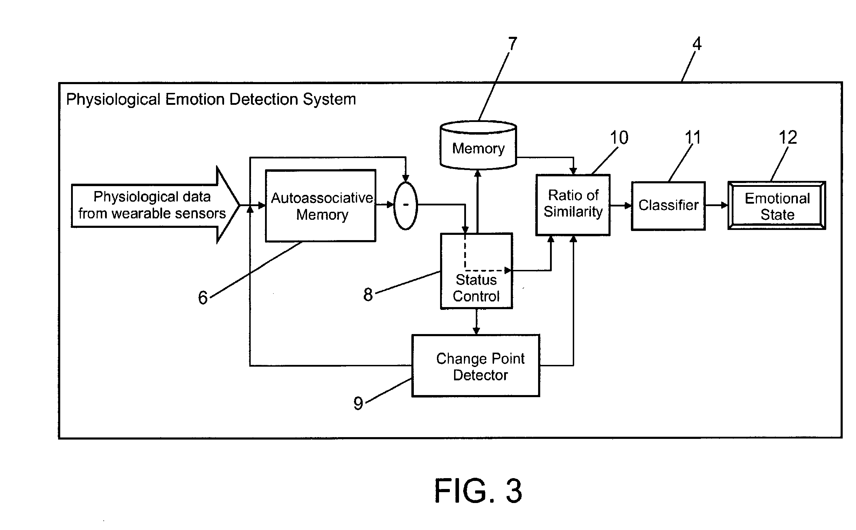

Figure 3 shows the Physiological Emotion Detection System 4 in further detail. It comprises an autoassociative memory (AM) 6, a memory 7 for conventional storage, status control 8, and a change point detector 9, whose combined work result in the computation of a ratio of similarity 10 (as described further in the present document). The ratio of similarity 10 is then used by a classifier 11 to finally determine the emotional state 12 of the user.

According to a preferred embodiment of the invention, the following steps are performed by the EC:

1) Organizing the response of the system to negative emotions using information entered by the user through options displayed on the embedded computer's touch screen (ECTS). Said response may include a change effected on a device located in the surroundings (e.g. switching the radio off), an action of an ambient electronic device (AED) (e.g. playing music), or a call to a relative, friend or professional carer.

2) Initiating continuous collection of data from a number of sensors measuring parameters of the autonomic nervous system. Said sensors are in contact with a person's body and transmit data wireless at a given sampling rate in a format that is readable by a computer program implemented on an EC. Different sensors systems providing measurement for heart rate, ECG, skin conductance, pulse and/or body temperature, electromyogram, and other are known to those skilled in the art.

3) Providing said physiological data continuously and in real time to a pre-trained autoassociative memory (AM). An AM is a multi-input/output computing model where every input has a corresponding output which aims to possess identical properties as said input. The AM is trained using any supervised connectionist model known to those skilled in the art, e.g. backpropagation, Hebb-like, etc. Training involves providing the AM with physiological data related to a state which is predominant when a person remains in absence of emotional stimulation, e.g. neutral. Said training which is performed offline is stopped when the outputs approximate the inputs within a certain error margin. Using the stored function parameters, an AM can provide estimations for new data.

4) Continuously calculating the residuals between the raw sensor data (inputs) and the estimations (outputs) of the pre-trained AM. Residuals are the absolute value of the arithmetic subtraction between inputs and outputs at every period of time determined by the sampling rate, e.g. every 1 second.

5) Performing a calibration process to calculate maximal and minimal residual values for each signal. Said calibration process is performed only upon first use and involves the AM and sensors operating for a certain period of time (from 30 seconds up to 2 hours) while the subject remains in semi-recumbent position. No indication of emotional states is provided during said calibration process.

6) Initiating regular operation once calibration is finished. At this point residual values are continuously accumulated to determine the moment a change from neutral to non-neutral emotional state takes place. The user can interrupt regular operation and instruct the system to operate the AEM or any other device to produce a response similar to that which follows the detection of a negative state. This action implies an Error of type II and increases a misrecognition counter (MRC) by 1.

7) Identifying the moment the accumulated residual values exceeds a given threshold. Residual values for all physiological signals acquired over the last N seconds before a change point is detected are kept in the embedded computers memory (ECM). Change point can be calculated on the residuals of one or more signals.

8) Calculating the ratio of similarity between the neutral and non-neutral emotional state. This is done using a sequential calculation of the ratio of difference between the last seconds of known neutral physiological data and subsequent incoming non-neutral physiological data. In other words, the block of data representing the psychophysiological condition before the non-emotional state was detected is held in the EC memory and compared to incoming data blocks of similar length. We call this a 'shuffling' comparison. A number of tools to estimate the ratio of similarity exists which are known to one skilled in the art, e.g. Euclidean distance, Jeffries-Matusita distance, PSD Ratio.

9) Categorizing the non-emotional state into a number of emotional classes using a vote-based classification method. This step is executed by providing the ratio of similarity of each input signal to a number of classification methods already trained to classify various emotional categories e.g. anger, sadness, fear, positive, negative, etc. The number of classification methods employed in this step should be an odd number that exceeds by 1 the number of emotional classes that are to be detected. The output from each of the classification method counts as a single vote towards a final decision about the emotion a person is experiencing. This is done until the change point calculation indicates a return to neutrality/normality.

10) Inquiring the user about their emotional well-being upon identification of a negative emotional state. When a negative emotional state has won the majority of votes from the classification methods, a message is displayed on the touch screen of the embedded computer (EC) with a text that makes reference to the emotional state of the person, e.g. 'It seems you are experiencing an intense negative emotion. Do you feel OK?', 'Do you need help?', 'is everything OK?', 'Do you feel emotional stressed?', etc.

11) Acquiring the user's response via the ECTS. The user is prompted to press the button on screen that best describes their current state e.g. 'yes', 'no', or 'I do not feel any negative emotion'.

12) Sending commands to an ambient electronic device when a negative emotion is detected and confirmed by the user.

13) Evaluating the accuracy of the system. If the answer selected by the user through ECTS does not match the system's output, i.e. the user is not experiencing a negative emotion (Error Type I), MRC is increased by 1.

14) Determining the need to adapt the AM based on MRC value. When the value of MRC exceeds a given threshold over a required period of time the detection of the emotional state is stopped. A process is simultaneously initiated including the steps to retrain the AM.

15) Resuming regular operation involving online evaluation of physiological data (from steps sixth through fourteenth).

In one embodiment of the present invention, an AM previously trained with data relating to the ECG and HR of six persons while they were in the neutral emotional state was used. The AM was trained using an iterative method known as back-propagation (BP) which stopped when the percentage of error between the original sensor data and the AM estimations fell below 5%. The trained AM, represented by a series of numerical weights and biases, is stored on the EC's memory along with all the instructions required to interact with the physiological sensors, respond to a negative emotion, and operate the AED. An exemplary EC is provided by HTC Corp. (Taiwan, RPC) and features a 624 MHz processor, 128 MB of RA memory, and connectivity through Bluetooth 2.0 and WiFi (IEEE 802.11 b/g) among other.

A wireless, wearable physiological sensor system 1 in the form of a 18 sq. cm. plastic box with two chest electrodes called AliveHeart Monitor available from Alive Technologies (Queensland, Australia) is used to collect data in real time. When activated this device sends information onto the EC 2 at a rate of 5 samples per second with a resolution of 300Hz for ECG. Additional embodiments can include other wearable sensing devices which collect physiological data and transmit said data remotely onto a computer.

In accordance with a preferred embodiment the programming that directs the EC's operation is implemented using C++ language on Windows Mobile Operating System available from Microsoft Corporation (Washington, USA). It is easily recognizable by one skilled in the art that various programming languages and techniques to implement the instructions that govern the EC operation can be used.

The EC is wirelessly connected to an ambient electronic device 3 called Nabaztag available from Violet (France) which is an apparatus in the shape of rabbit which is capable of wirelessly receiving voice and text commands as well as messages via WiFi. In other embodiments AEDs such as Chumby from Chumby industries (San Diego, USA), Mist from Ambient Devices (Cambridge, USA), or any alternative device capable of being remotely controlled, e.g. a desk lamp can also be utilized using similar principles to those disclosed in the present invention.

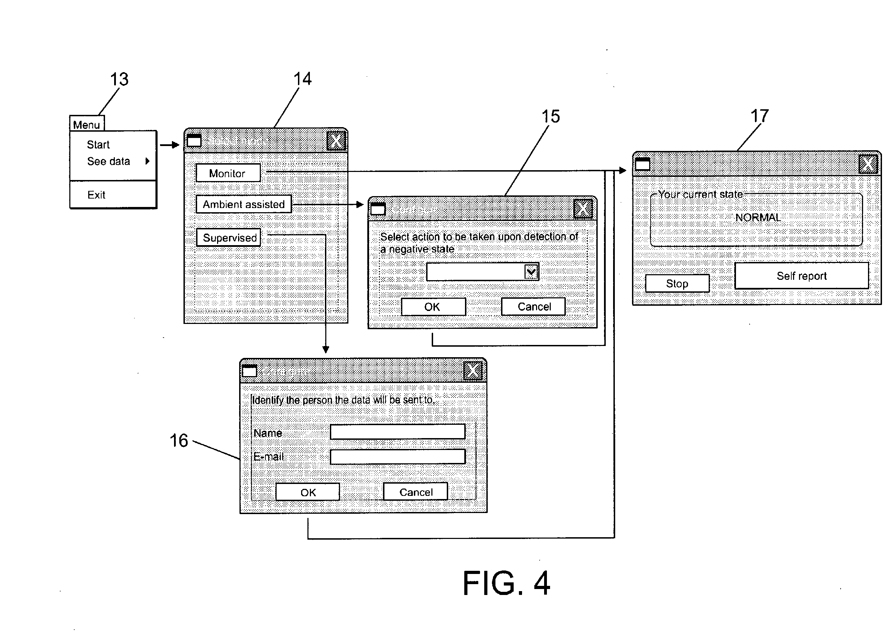

As shown in figure 4, upon system startup 13 a first configuration window 14 is displayed on ECTS which allows the user to choose between three forms of operation: 'monitorization', 'interactive', or 'automatic'. Each of these options affects the way the system responds to a confirmation from the user about the existence of a negative emotion. The 'monitorization' mode involves no action subsequent to the appearance of the negative emotional state. The 'interactive' option will send a series of HTML commands to the AED to initiate an action.

Additionally, an 'interactive' operation requires the user to enter further information in relation to the response of the AED to a negative emotional state in a second configuration window 15, such as: 'switch the light on', 'switch the radio on', 'read the content of URL address:', and 'play a voice message'. The latter two parameters need to be complemented with a URL address or a text message which often cannot exceed a number of characters specified by the AED manufacturer.

'Automatic' operation will send a message to a remote location using electronic mail. Thus, 'Automatic' operation requires the user to enter an email address in a third configuration window 16 which will become the recipient of a message informing about the negative emotional state that is being experienced by the user. In another embodiment the user replaces the action of sending a message to an email account with the possibility of making a phone call to a person whose number is typed on the ECTS or stored on the EC's memory. This can be done using the EC's own GSM capabilities which are controlled by the EC's program. Yet in another embodiment, VoIP (voice over internet protocol) applications such as Skype (available from Skype Limited, Luxemburg, Luxemburg) or Googletalk (available from Google, California, USA) may also be employed.

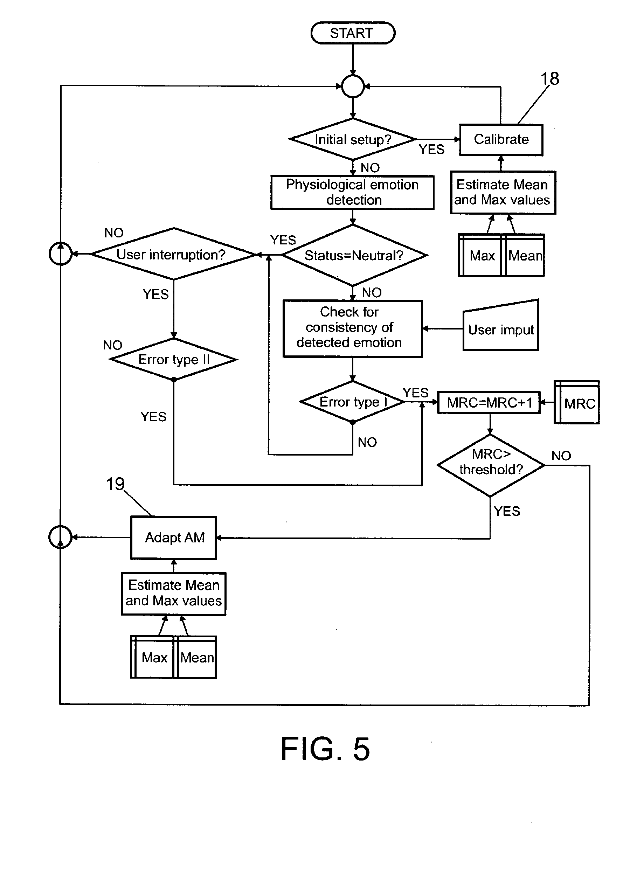

When the user exits the configuration windows, a calibration process 18 is started with the intention of adjusting the AM parameters to the specific physical characteristics of the current user. This calibration process 18 as well as the long term adaptation 19 of the AM is indicated in Figure 5. This process is executed on the EC only once for every new user or when the user deems it appropriate (after very long periods of system activity for instance). Note that calibration 18 does not involve changes on the parameters of the trained AM but only calculation of the mean residual values and threshold used in the detection of a change point. Once calibration is finished, the system initiates regular operation which is indicated by a monitorization window 17 on the ECSC containing the words 'Normal'. Said monitorization window includes a button that enables the user to indicate the occurrence of a negative emotional state that went undetected by the system.

Data coming from the sensors is continuously provided to the previously trained AM. Residuals are then calculate for each data sample of the two aforementioned signals, ECG and HR, and accumulated over time. The accumulated value is then evaluated using a method of change point detection. Note that at all times the last 4 seconds of information are kept in the computer's memory.

In one embodiment of the present invention a preferred method to detect change point called non-parametric cumulative sum (NPCUSUM) is used. A different embodiment may employ any truncated or open ended non-parametric change point detection method known to those skilled in the art, e.g. exponential smoothing.

```
 Start monitoring.
For each sample n of physiological signal PhS calculate
      Z(n)= Residual(PhS(n))-C;
      CUSUM=CUSUM+Z(n);
      If CUSUM<0
          CUSUM=0;
      Else
      CUSUM=CUSUM;
      Endif
      If CUSUM>dThreshold
          Class='NonNeutral'
          CUSUM=0;
      Else
          Class='Neutral';
      Endif
```

Where

Residual(PhS(n)) calculates the difference between estimated and real
values of physiological data PhS;
$Z(n)$ is the residual value for current sample n of PhS shifted by the value of C.
CUSUM is the value of the NP cumulative sum initially set to 0 (after calibration);
SampleR is the wearable sensors sampling rate (1/Hertz);
Mean is the mean value of PhS residual values calculated during calibration or adaptation;
MDD is the maximal detection time in seconds (5 in this particular case);
T is the maximal detection delay in number of samples (MDD/SampleR);
C is a shifting constant which can take the absolute value of (Mean)*2;
Class is the emotional state identified by the system (Neutral, Nonneutral);
MaxValue is the Maximum value of the residual calculated on data from
a neutral state during training;
dThreshold is the detection threshold (T * (MaxValue-C)).

So long as CUSUM does not become larger than dThreshold the ECTS will show a message indicating a normal state.

On the other hand when the accumulated residual value exceeds dThreshold, a shuffling comparison is started whereby the ratio of similarity between the last 4 seconds of physiological data before the change point is detected and incoming 4-second blocks of physiological data is calculated. Said shuffling comparison is implemented in accordance with the following algorithm:

```
If Class= 'NonNeutral'
    Retrieve NE from memory;
    Collect NNE;
    Calculate Ratio;
    Perform classification using Ratio Values;
    Resume Regular Operation;
Else
    Continue Regular Operation;
Endif
```

Where:

NE is a vector of n samples related to the last X seconds of data before a change point was detected (in this embodiment X=4);
NNE is a vector of n samples related to the X seconds of data after the change point was detected (in this embodiment X=4);
Ratio is the ratio of similarity between NE and NNE.

One embodiment involved the calculation of the ratio of similarity between the power spectral density (PSD) of the four seconds of physiological information prior to change point and all subsequent blocks of 4 seconds of physiological data. The ratio is estimated using the below algorithm:

$$Ratio = ( 2 * \log(NEd+NNEd) - \log(4) - \log(NEd) - \log(NNEd) )$$

Where:

NEd is the Power Spectral Density (PSD) of NE;
NNEd is the PSD of NNE.

In another embodiment the Euclidean distance is estimated on data blocks using the below algorithm:

$$Ratio = \sum_{P=1}^{n} \sqrt{\left(NE(p) - NNE(p)\right)^2}$$

Where:

n is the number of samples contained in 4 seconds.

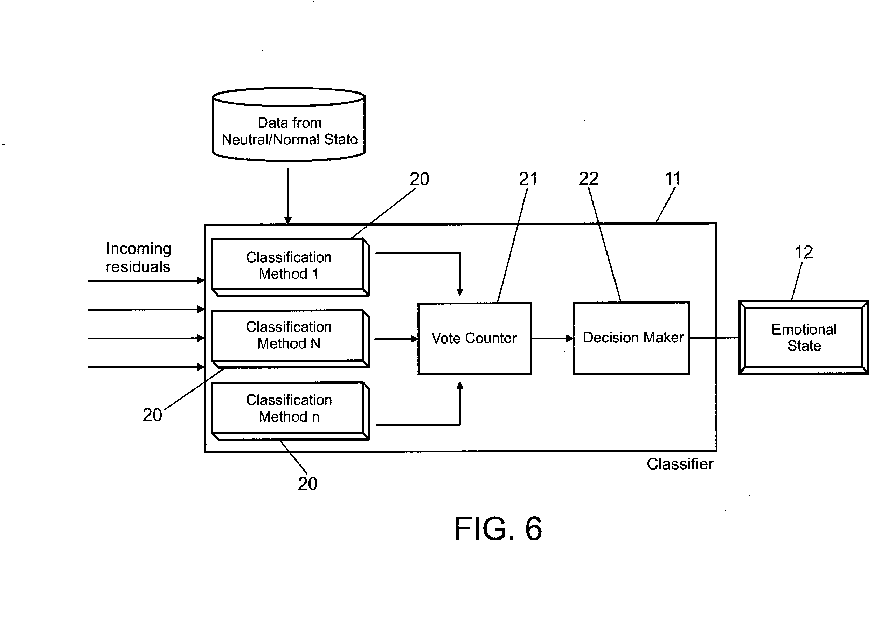

As shown in figure 6, the ratio calculated for each of the physiological signals is fed into a plurality of classifications methods 20 which in this preferred embodiment are: 1) Support Vector Machines with a polynomial algorithm of second order, 2) linear discriminant analysis (LDA), and 3) decision tree with a minimum size of split of 10 and minimum size of leaves of 1. Said methods having been previously trained with data associated with the residuals of the classes of negative and positive emotions.

The result from each method, positive or negative, counts as a vote towards the final classification of the emotional class (being these votes computed by a vote counter 21). Therefore, the class with the majority of votes is chosen by a decision maker 22 as the result of the detection procedure.

If the result points to a negative emotion, a message appears on the ECTS asking the user to respond to the following question: "It seems you are in the middle of a problematic situation. Are you emotionally stressed?'. The user can choose one of two possible answers: 'yes', 'No, I do not feel any negative feeling". If the answer from the user does not correspond with that of the system, i.e., the user is not experiencing experiencing a negative emotion (error Type I), the current MRC value is increased by one. The system registers the time an MRC occurs in order to quantify their frequency. A subsequent validation determines whether the MRC frequency exceeds MRCThreshold. If this is true, the system automatically initiates an adaptation 19 of AM using the back propagation algorithm described above. This process additional involves recalculation of the mean residual values used in NPCUSUM estimations. During this time the system produces no emotional output. Note that MRC is also increased when the user interrupts regular operation (Error Type II).

AM adaptation is performed in accordance with the below algorithm.

```
If (MRC > MRCThreshold)
    While (Residual(PhS(n)) > Error OR TimeOUT)
        RetrainNetWeigths(Residual(PhS(n))),
    TrainningCoeff;
    End while;
    Recalculate Mean and Max for the newly trained AM;
End if;
```

Where

MRCThreshold is a threshold previously set for maximum admitted frequency of MRCs. This is calculated by the maximal allowed number of occurrences of Errors Type I and II divided by a period of time in seconds, e.g. 10 MRCS in 7200 seconds;

Error is a value close to zero which represents the desired maximal difference between actual and estimated values of sample n from PhS (residual value);

TimeOUT is the flag for maximum training time;

TrainingCoeff establishes the training coefficient according to the type of error produced. Said coefficient is increased according to the error's type and can take any value between from 0 and 1 where 0 means adaptation of current weights and 1 implies regeneration of all AM weights. TrainingCoeff is thus related to the length and duration of AM's re-training;

RetrainNetWeigths modifies the AM in accordance with TrinningCoeff.

Once adaptation finishes the algorithm resumes normal operation.

If the user responds 'yes' to the occurrence of a negative emotion, the system produces the output associated with the previously selected operation mode ('Monitorization', 'Interactive' or 'Automatic') and then resumes monitoring.

**Claims**

1.  Affective well-being supervision system comprising:

    - at least one wearable sensor (1) configured to measure at least one physiological signal of a user;
    - logical means (2) configured to receive the at least one measured physiological signal, to detect an emotional change according to said signal, and to generate instructions to an external device (3) according to the detected emotional change;
    - first communication means configured to connect the at least one wearable sensor (1) and the logical means (2);
    - second communication means configured to connect the logical means (2) to the external device (3) and to send the generated instructions to said external device (3); **characterized in that** the logical means (2) comprise a memory (7) and **in that** the logical means (2) are adapted to:
    - if a data block of the measured signal is classified as being a neutral physiological state, store in the memory (7) said data block classified as being a neutral physiological state and;
    - detect the emotional change by comparing each incoming data block of the measured physiological signal with the last stored in the memory (7) data block of said signal classified as being a neutral physiological state.

2.  System according to claim 1 wherein the logical means further comprise a non parametric sequential change point detector (9).

3.  System according to claim 1 wherein the logical means further comprise a plurality of classifying methods (20) sharing a same input, and a vote counter (21) that determines if the emotional change occurs according to outputs of said plurality of classifying methods (20).

4.  System according to claim 3 wherein the plurality of classifying methods (20) comprise a Support Vector Machine, a Linear discriminant analysis, and a decision tree.

5.  System according to any of the previous claims wherein the first communication means are wireless communication means.

6.  System according to any of the previous claims wherein the second communication means are wireless communication means.

7.  System according to any of the previous claims wherein the logical means (2) comprise a previously trained autoassociative memory (6) and an accumulator to compute and accumulate a difference between the measured signal and an estimation of said measured signal performed by the autoassociative memory (6).

8.  System according to claim 7 wherein the logical means (2) further comprise a misrecognition counter which computes a number of false emotional changes detections, and wherein the logical means (2) are configured to train the autoassociative memory (6) if the misrecognition counter exceeds a threshold.

9. Affective well-being supervision method comprising:

- measuring at least one physiological signal of a user,
- detecting an emotional change according to the measured signal;
- generating instructions to an external device (3) according to the detected emotional change;
**characterized in that** the step of detecting the emotional change further comprises:
- if a data block of the measured signal is classified as being a neutral physiological state, storing in the memory (7) said data block classified as being a neutral physiological state;
- and comparing each incoming data block of the measured physiological signal with the last stored in the memory (7) data block of said physiological signal classified as being a neutral physiological state.

10. Method according to claim 9 wherein the step of detecting the emotional change further comprises applying a non parametric sequential change point detector (9).

11. Method according to any of claims 9 to 10 wherein the step of detecting the emotional change further comprises applying a plurality of classifying methods (20) sharing a same input, and using outputs of said plurality of classifying methods (20) in a vote counter (21) that determines if the emotional change occurs.

12. Method according to claim 11 wherein the plurality of classifying methods (20) comprise a Support Vector Machine, a Linear discriminant analysis, and a decision tree.

13. Method according to any of claims 9 to 12 wherein the step of detecting an emotional change according to the measured signal comprises estimating the measured signal by means of a previously trained autoassociative memory (6) and computing a difference between the measured signal and the estimation of said measured signal.

14. Method according to claim 13 further comprising, if the computed difference exceeds a threshold, comparing a segment of the at least one measured signal and a previously stored segment of a signal corresponding to a reference emotional state.

15. Method according to any of claims 13 and 14 further comprising computing a number of false emotional changes detections, and training the autoassociative memory (6) if the misrecognition counter exceeds a threshold.

## Patentansprüche

1. System zur Überwachung von emotionalem Wohlbefinden, aufweisend:

- mindestens einen tragbaren Sensor (1), der ausgebildet ist, um mindestens ein physiologisches Signal eines Benutzers zu messen;
- logische Mittel (2), die dazu konfiguriert sind, um mindestens ein gemessenes physiologisches Signal zu empfangen, um eine emotionale Veränderung aufgrund des genannten Signal zu erfassen, und um Anordnungen an ein externes Gerät (3) entsprechend der erfassten emotionalen Veränderung zu erzeugen;
- erste Kommunikationsmittel, die dazu konfiguriert sind, den mindestens einen tragbaren Sensor (1) mit den logischen Mitteln (2) zu verbinden;
- zweite Kommunikationsmittel, die dazu konfiguriert sind, die logischen Mittel (2) mit dem externen Gerät (3) zu verbinden und die erzeugten Anordnungen zu dem genanntem externen Gerät (3) zu senden;
**dadurch gekennzeichnet, dass** die logischen Mittel (2) einen Speicher (7) aufweisen und dass die logischen Mittel (2) ausgestaltet sind,
- dass, falls ein Datenblock des gemessenen Signals als neutraler physiologischer Zustand eingestuft wird, dieser genannte Datenblock, der als neutraler physiologischer Zustand eingestuft wird, in dem Speicher (7) gespeichert wird,
- und dass sie die emotionale Veränderung erfassen, indem sie jeden einkommenden Datenblock des gemessenen physiologischen Signals mit dem zuletzt im Speicher (7) gespeichertem Datenblock des als neutraler physiologischer Zustand eingestuftem Signals vergleichen.

2. System nach Anspruch 1, wobei die logischen Mittel weiter einen nichtparametrischen, sequentiellen Veränderungspunktdetektor (9) aufweisen.

**3.** System nach Anspruch 1, wobei die logischen Mittel ferner eine Vielzahl von Klassifizierverfahren (20) aufweisen, die einen gleichen Input teilen, sowie einen Stimmenzähler (21), der ermittelt, ob gemäß dem Ergebnis der genannten Vielzahl von Klassifizierverfahren (20)eine emotionale Veränderung stattfindet.

**4.** System nach Anspruch 3, wobei die Vielzahl von Klassifizierverfahren (20) eine Stützvektormaschine, eine lineare Diskriminanzanalyse und einen Entscheidungsbaum aufweist.

**5.** System nach einem der vorangegangenen Ansprüche, wobei die ersten Kommunikationsmittel drahtlose Kommunikationsmittel sind.

**6.** System nach einem der vorangegangenen Ansprüche, wobei die zweiten Kommunikationsmittel drahtlose Kommunikationsmittel sind.

**7.** System nach einem der vorangegangenen Ansprüche, wobei die logischen Mittel (2) einen zuvor trainierten, autoassoziativen Speicher (6) und einen Akkumulator aufweisen, um die Differenz zwischen dem gemessenem Signal und einer Schätzung des genannten Signals, die von dem autoassoziativen Speicher (6) durchgeführt wurde, zu berechnen und sammeln.

**8.** System nach Anspruch 7, wobei die logischen Mittel (2) des Weiteren einen Erkennungsfehlerzähler beinhalten, der eine Anzahl von falschen emotionalen Veränderungsnachweisen berechnet, und wobei die logischen Mittel (2) dazu ausgebildet sind, den autoassoziativen Speicher (6) zu trainieren, wenn der Erkennungsfehlerzähler einen Grenzwert überschreitet.

**9.** Verfahren zur Überwachung von emotionalem Wohlbefinden, mit folgenden Merkmalen:

- Messen von mindestens einem physiologischen Signal eines Benutzers;
- Erfassen einer emotionalen Veränderung gemäß des gemessenen Signals;
- Erzeugen von Anweisungen an ein externes Gerät (3) entsprechend der erfassten emotionalen Veränderung; **dadurch gekennzeichnet, dass** der Schritt zur Erfassung der emotionalen Veränderung ferner vorsieht:
- dass, falls ein Datenblock des gemessenen Signals als neutraler physiologischer Zustand eingestuft wird, dieser besagte Datenblock, der als neutraler physiologischer Zustand eingestuft wurde, im Speicher (7) gespeichert wird;
- und dass jeder einkommende Datenblock des gemessenen physiologischen Signals mit dem zuletzt im Speicher (7) gespeichertem Datenblock des besagten physiologischen Signals verglichen wird, der als neutraler physiologischer Zustand eingestuft wurde.

**10.** Verfahren nach Anspruch 9, wobei der Schritt zur Erfassung der emotionalen Veränderung des Weiteren die Anwendung eines nichtparametrischen sequentiellen Veränderungspunktdetektors (9) beinhaltet.

**11.** Verfahren nach einem der Ansprüche 9 bis 10, wobei der Schritt zur Erfassung der emotionalen Veränderung ferner die Anwendung einer Vielzahl von Klassifizierverfahren (20) umfasst, die einen gleichen Input benutzen, sowie die Benutzung der Ergebnisse der besagten Vielzahl von Klassifizierverfahren (20) in einem Stimmenzähler (21), der ermittelt, ob eine emotionale Veränderung stattfindet.

**12.** Verfahren nach Anspruch 11, wobei die Vielzahl von Klassifizierverfahren (20) eine Stützvektormaschine, eine lineare Diskriminanzanalyse und einen Entscheidungsbaum aufweist.

**13.** Verfahren nach einem der Ansprüche 9 bis 12, wobei der Schritt zur Erfassung einer emotionalen Veränderung gemäß dem gemessenem Signal das Schätzen des gemessenen Signals durch einen zuvor trainierten, autoassoziativen Speicher (6) umfasst und das Berechnen der Differenz zwischen dem gemessenem Signal und der Schätzung des besagten Signals.

**14.** Verfahren nach Anspruch 13, wobei weiter vorgesehen ist, dass, falls die berechnete Differenz einen Grenzwert überschreitet, ein Segment von mindestens einem gemessenem Signal mit einem zuvor gespeichertem Segment eines Signals verglichen wird, das einem emotionalen Referenzzustand entspricht.

**15.** Verfahren nach einem der Ansprüche 13 und 14, ferner umfassend ein Berechnen einer Anzahl von falschen emotionalen Veränderungsnachweisen und das Trainieren des autoassoziativen Speichers (6), falls der Erkennungsfehlerzähler einen Grenzwert überschreitet.

## Revendications

**1.** Système de supervision du bien-être affectif comprenant :

- au moins un détecteur portatif (1) configuré pour mesurer au moins un signal physiologique d'un utilisateur ;
- des moyens logiques (2) configurés pour recevoir l'au moins un signal physiologique mesuré, pour détecter un changement émotionnel conformément audit signal et pour générer des instructions vers un dispositif externe (3) conformément au changement émotionnel détecté ;
- des premiers moyens de communication configurés pour connecter l'au moins un détecteur portatif (1) et les moyens logiques (2) ;
- des seconds moyens de communication configurés pour connecter les moyens logiques (2) au dispositif externe (3) et pour envoyer les instructions générées audit dispositif externe (3) ;
**caractérisé en ce que** les moyens logiques (2) comprennent une mémoire (7) et **en ce que** les moyens logiques (2) sont adaptés pour :
- si un bloc de données du signal mesuré est classé comme étant un état physiologique neutre, stocker dans la mémoire (7) ledit bloc de données classé comme étant un état physiologique neutre ; et
- détecter le changement émotionnel en comparant chaque bloc de données entrant du signal physiologique mesuré avec le dernier bloc de données stocké dans la mémoire (7) dudit signal classé comme étant un état physiologique neutre.

**2.** Système selon la revendication 1, dans lequel les moyens logiques comprennent en outre un détecteur de point de changement séquentiel non paramétrique (9).

**3.** Système selon la revendication 1, dans lequel les moyens logiques comprennent en outre une pluralité de méthodes de classement (20) partageant une même entrée, et un compteur de voix (21) qui détermine si le changement émotionnel survient conformément aux sorties de ladite pluralité de méthodes de classement (20).

**4.** Système selon la revendication 3, dans lequel la pluralité de méthodes de classement (20) comprend une machine à vecteurs de support, une analyse discriminante linéaire et un arbre de décision.

**5.** Système selon l'une quelconque des revendications précédentes, dans lequel les premiers moyens de communication sont des moyens de communication sans fil.

**6.** Système selon l'une quelconque des revendications précédentes, dans lequel les seconds moyens de communication sont des moyens de communication sans fil.

**7.** Système selon l'une quelconque des revendications précédentes, dans lequel les moyens logiques (2) comprennent une mémoire auto-associative préalablement paramétrée (6) et un accumulateur pour calculer et accumuler une différence entre le signal mesuré et une estimation dudit signal mesuré réalisée par la mémoire auto-associative (6).

**8.** Système selon la revendication 7, dans lequel les moyens logiques (2) comprennent en outre un compteur de reconnaissances erronées qui calcule un nombre de fausses détections de changements émotionnels, et dans lequel les moyens logiques (2) sont configurés pour paramétrer la mémoire auto-associative (6) si le compteur de reconnaissances erronées dépasse un seuil.

**9.** Procédé de supervision du bien-être affectif comprenant :

- la mesure d'au moins un signal physiologique d'un utilisateur ;
- la détection d'un changement émotionnel conformément au signal mesuré ;
- la génération d'instructions vers un dispositif externe (3) conformément au changement émotionnel détecté ;
**caractérisé en ce que** l'étape de détection du changement émotionnel comprend en outre :
- si un bloc de données du signal mesuré est classé comme étant un état physiologique neutre, le stockage dans la mémoire (7) dudit bloc de données classé comme étant un état physiologique neutre ;

- et la comparaison de chaque bloc de données entrant du signal physiologique mesuré avec le dernier bloc de données stocké dans la mémoire (7) dudit signal classé comme étant un état physiologique neutre.

10. Procédé selon la revendication 9, dans lequel l'étape de détection du changement émotionnel comprend en outre l'application d'un détecteur de point de changement séquentiel non paramétrique (9).

11. Procédé selon l'une quelconque des revendications 9 à 10, dans lequel l'étape de détection du changement émotionnel comprend en outre l'application d'une pluralité de méthodes de classement (20) partageant une même entrée, et l'utilisation de sorties de ladite pluralité de méthodes de classement (20) dans un compteur de voix (21) qui détermine si le changement émotionnel survient.

12. Procédé selon la revendication 11, dans lequel la pluralité de méthodes de classement (20) comprend une machine à vecteurs de support, une analyse discriminante linéaire et un arbre de décision.

13. Procédé selon l'une quelconque des revendications 9 à 12, dans lequel l'étape de détection d'un changement émotionnel conformément au signal mesuré comprend l'estimation du signal mesuré au moyen d'une mémoire auto-associative préalablement paramétrée (6) et le calcul d'une différence entre le signal mesuré et l'estimation dudit signal mesuré.

14. Procédé selon la revendication 13, comprenant en outre, si la différence calculée dépasse un seuil, la comparaison d'un segment de l'au moins un signal mesuré et d'un segment préalablement stocké d'un signal correspondant à un état émotionnel de référence.

15. Procédé selon l'une quelconque des revendications 13 et 14, comprenant en outre le calcul d'un nombre de fausses détections de changements émotionnels, et le paramétrage de la mémoire auto-associative (6) si le compteur de reconnaissances erronées dépasse un seuil.

**FIG. 1**

FIG. 2

FIG. 3

FIG. 4

EP 2 515 760 B1

FIG. 5

FIG. 6

**EP 2 515 760 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003139654 A
- US 6656116 B2
- US 6190314 B1
- US 5507291 A
- US 2008221401 A
- US 5601090 A
- US 5676138 A
- WO 2008129356 A
- US 6609024 B
- US 6021346 A
- US 20070192108 A
- WO 2008028391 A
- JP 2005237668 A
- US 20070142732 A
- US 5974262 A
- US 4683891 A
- US 5741217 A
- US 5682803 A
- US 6026322 A
- US 20070167850 A
- WO 2009037612 A2
- WO 2006009830 A2
- US 2009253996 A